Europäisches Patentamt

European Patent Office

Office européen des brevets

(19)

(11) **EP 0 815 458 B1**

(12) **EUROPEAN PATENT SPECIFICATION**

(45) Date of publication and mention
of the grant of the patent:
**29.10.2003 Bulletin 2003/44**

(51) Int Cl.7: **G01R 27/02**

(86) International application number:
**PCT/US96/03604**

(21) Application number: **96906675.2**

(22) Date of filing: **14.03.1996**

(87) International publication number:
**WO 96/028741 (19.09.1996 Gazette 1996/42)**

(54) **DETERMINING THE DIELECTRIC PROPERTIES OF WOOD**

BESTIMMUNG DER DIELEKTRISCHEN EIGENSCHAFTEN VON HOLZ

DETERMINER LES PROPRIETES DIELECTRIQUES DU BOIS

(84) Designated Contracting States:
**AT BE CH DE DK ES FI FR GB GR IE IT LI LU MC NL PT SE**
Designated Extension States:
**AL LT LV SI**

(30) Priority: **15.03.1995 ZA 9502126**

(43) Date of publication of application:
**07.01.1998 Bulletin 1998/02**

(73) Proprietors:
• **Venter, Liebrecht Rudolph**
  **Stellenbosch 7600 (ZA)**
• **viljoen, Jacobus Petrus Septimus**
  **Stellenbosch 7600 (ZA)**

(72) Inventors:
• **VENTER, Liebrecht, Rudolph**
  **Stellenbosch 7600 (ZA)**
• **VILJOEN, Jacobus, Petrus, Septimus**
  **Stellenbosch 7600 (ZA)**

(74) Representative: **Glaeser, Joachim, Dipl.-Ing. et al**
**Patentanwälte**
**DIEHL GLAESER HILTL & PARTNER**
**Königstrasse 28**
**22767 Hamburg (DE)**

(56) References cited:
**US-A- 3 593 128**          **US-A- 3 778 707**
**US-A- 3 992 665**          **US-A- 4 107 599**
**US-A- 4 174 498**          **US-A- 4 181 881**

**EP 0 815 458 B1**

**Description**

This invention relates to a method of determining the dielectric properties of wood, in particular for purposes of deriving therefrom a measure of the moisture content of the wood, and to means for use in such method.

The moisture content of wood can be expressed as the difference between the wet weight and the dry weight of the wood, divided by the dry weight.

When drying wood in a wood drying kiln, an end-point moisture content of 5% to 20% is normally required. Traditional methods of measuring the moisture content of wood, whilst reasonably accurate towards the end-point moisture content, become less accurate at higher values of the moisture content. At a moisture contents of above 30%, the traditional methods become completely unreliable.

For the proper control of the environment in which wood is dried, for example, in a wood-drying kiln, it is important for the moisture content of the wood to be known accurately while the moisture content is still relatively high, e.g. above 30%. If the moisture content is accurately known at these relatively high values it becomes possible to accelerate the drying process considerably, without causing undue stresses int the wood.

A method of determining the dielectric properties of wood is known from US-A-4 174 498. In particular, wood chips are disposed between electrodes and an electrical signal is applied thereto. An output of a measurement circuit provides a signal which is representative of the electrical impedance of the wood chips between the electrodes.

A wood-drying kiln in combination with means for determining dielectric properties thereof is known from US-A-4 107 599.

According to the present invention there is provided a method according to claim 1.

The varying electrical signal can conveniently be a sinusoidal voltage and can be applied to the electrodes via a resistive element, and the phase angle between the applied voltage (i.e. the voltage before the resistive element) and the voltage across the electrodes (i.e. the voltage after the resistive element), and the magnitudes of said voltages determined. From this it is possible to derive the phase angle and magnitude of the complex impedance between the electrodes.

It will be understood that one of the electrodes may be ground, i.e. the structure on which the wood is supported, where the structure is of an electrically conductive material.

The measured values may be determined in an electronics module which is in close proximity to the electrodes, and the phase angle and magnitude of said complex impedance may be determined from the measured values in data processing means which is remote from the electrodes, there being a data link between the electronics module and the data processing means.

Further according to the invention there is provided means for determining the dielectric properties of wood according to claim 4.

Still further according to the invention there is provided a wood-drying installation which comprises a wood-drying kiln, according to claim 5.

The invention will now be described in more detail, by way of example, with reference to the accompanying diagrammatic drawings.

In the drawings:

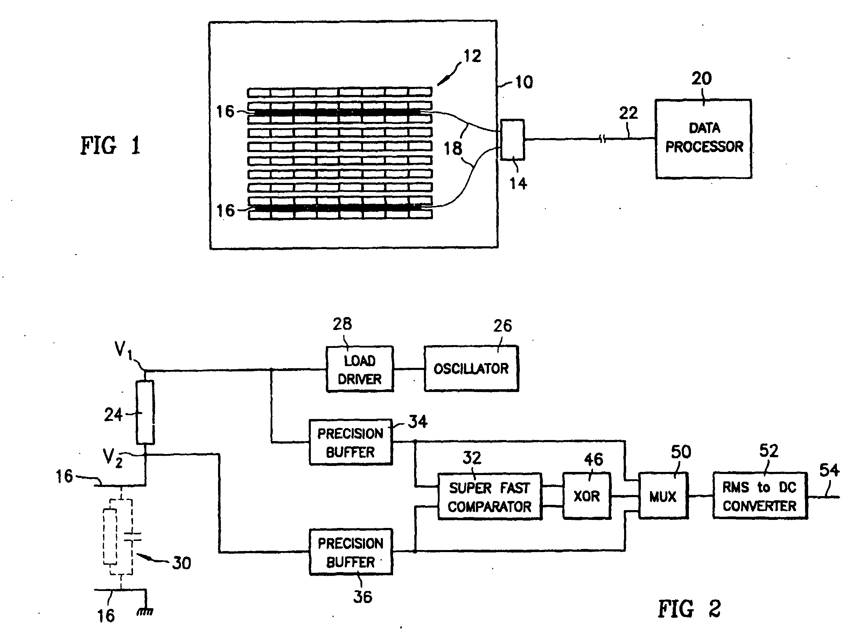

Figure 1 is an end view of a stack of timber, in a wood drying kiln provided with measuring means in accordance with the invention;
Figure 2 is a block diagram of the measuring means;
Figure 3 shows certain voltage waveforms; and
Figure 4 is a phasor diagram of the voltages.

Referring now the drawings in more detail, reference numeral 10 indicates a wood-drying kiln in which there is a stack of timber 12. The timber 12 is arranged in layers which are spaced from one another by means of spacers. The environment inside the kiln is controlled according to the moisture content of the timber.

To determine the moisture content of the timber accurately, the kiln is provided with measuring means comprising an electronics module 14 outside but in close proximity to the kiln, a pair of electrodes 16 inside the kiln and coupled to the module 14 by means of electrical connections 18, and a remote data processor 20 which is connected to the electronics module 14 by means of a data link 22. Where there are a number of kilns 10, each with its own electronics module 14, the various electronics modules may all be connected to the same data processor 20.

The electrodes 16 are in the form of metal plates and are simply inserted into the spaces between layers in the stack 12. It is an important feature of the invention that the electrodes 16 need not be cleaned prior to insertion into the stack, as operation of the apparatus is not affected by the degree of physical contact with the timber. The size and exact position of the electrodes is also not important. If the size and/or position of the electrodes is changed, all that will be required is for the system to be recalibrated.

The electronics module 14 serves to measure the values that are required to determine the phase angle and magnitude of the complex impedance between the electrodes 16. This is achieved in the following manner. The electronics module 14 includes a resistive element 24, connected in series with one of the electrodes, and an oscillator 26 and associated driver 28 whereby a sinusoidal voltage can be applied to the electrodes via the resistive element. The oscillator 26 has a frequency which is in the ultra-sonic range, for example in the order of 40kHz. The impedance indicated at 30 represents the impedance between the electrodes 16.

The electronics module 14 further comprises a super-fast comparator 32 which is connected via a precision buffer 34 to the output of the driver 28, and via a precision buffer 36 to one of the electrodes, the other electrode being connected to ground.

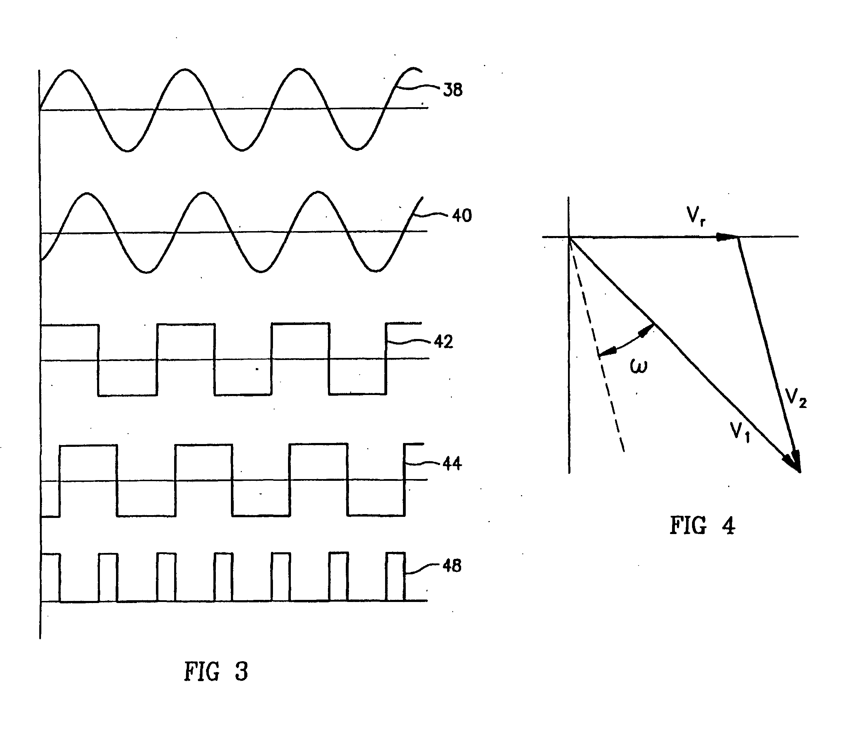

The waveform of the applied voltage (i.e. the output of the driver 28) is indicated at 38 in Figure 3, whereas the waveform of the voltage across the electrodes 16 (i.e. after the resistive element 24) is indicated by reference numeral 40 in Figure 3. In Figure 4 the applied voltage 38 is indicated by the phasor $V_1$ and the voltage across the electrodes by the phasor $V_2$. Because the impedance 30 is a complex impedance, there is a phase difference between the voltages $V_1$ and $V_2$, this being indicated by the angle $\omega$ in Figure 4. $V_r$ in Figure 4 is the voltage across the resistive element 24.

The comparator 32 serves to convert the sinusoidal voltages 38 and 40 to square-wave voltages 42 and 44 respectively.

The electronics module 14 further comprises an exclusive OR (XOR) circuit 46 whose output is indicated at 48 in Figure 3. The RMS value of the output 48 varies in proportion to the phase difference between the voltages 38 and 40. The outputs of the buffers 34 and 36 and the output of the XOR circuit 46 are fed via a multiplexer 50 to an RMS-to-DC converter 52. The multiplexer 50 has a relatively slow sampling rate as compared with the frequency of the applied signal.

Output 54 of the RMS-to-DC converter 52 is relayed to the data processor 20 via the data link 22.

The phase angle of the impedance 30 is determined by making use of the following equations:

$$a = V_2 * \cos \omega$$

$$b = V_2 * \sin \omega$$

$$d = V_1 / \{-a*a/b - b\}$$

$$c = a*d/b$$

$$C_x = d/\{R*2\pi f\}$$

$$R_x = R/(c-1)$$

Where:

$V_1$    is the amplitude of the applied voltage;
$V_2$    is the amplitude of the voltage across the electrodes;
$\omega$    is the phase difference between the voltages $V_1$ and $V_2$;
R    is the series resistive element 24;
f    is the frequency of the applied voltage;
$C_x$    is the value of the capacitive component of the complex impedance 30; and
$R_x$    is the resistive component of the complex impedance 30.

assuming that the capacitive and a resistive components of the impedance are in parallel.

In the event that the complex impedance includes an inductive component ($L_x$) in parallel with the capacitive

component $C_x$, the value of $L_x$ can be determined independently from $C_x$ by measuring the complex impedance at two different frequencies.

The moisture content of the timber is derived by suitable data processing in the data processor 20.

**Claims**

1. A method of determining the dielectric properties of wood i.e. its complex impedance comprised of the true or pure resistance $R_x$ and the true or pure capacitance $C_x$ of the wood as complex dielectric, which comprises disposing the wood (12) between electrodes (16), applying a varying signal of specified voltage to the electrodes via a resistive element (24) connected in series with one of the electrodes (16), measuring both the amplitude of the applied voltage and the amplitude of the voltage across the electrodes, and determining by the measured amplitude of the applied voltage, the amplitude of the voltage accross the electrodes and the angle representing the phase difference between these signals, the dielectric properties $R_x$ and $C_x$ of the wood disposed between the electrodes by use of the following equations, assuming that the capacitive and resistive components of the complex impedance (30) are in parallel:

$$a = V_2 \cos \,! \qquad (1)$$

$$b = V_2 \sin \,! \qquad (2)$$

$$d = \frac{V_1}{(-a(\frac{a}{b})-b)} \qquad (3)$$

$$c = \frac{ad}{b} \qquad (4)$$

$$C_x = \frac{d}{2\,fr\Pi} \qquad (5)$$

$$R_x = \frac{R}{(Hc-1)} \qquad (6)$$

where:

$V_1$ is the amplitude of the applied voltage;
$V_2$ is the amplitude of the voltage across the electrodes;
! is the phase difference between the voltages $V_1$ and $V_2$,
R is the series resistive element (24);
f is the frequency of the applied voltage;
$C_x$ is the value of the capacitive component of the complex impedance (30); and
$R_x$ is the resistive component of the complex impedance (30).

2. A method as claimed in claim 1, wherein the varying voltage is a sinusoidal voltage (38) applied to the electrodes (16) via the resistive element (24).

3. A method as claimed in claim 2, wherein the measured amplitudes of $V_1$ and $V_2$ and the phase difference between $V_1$ and $V_2$ are determined in an electronics module (14) which is in close proximity to the electrodes, and wherein the phase angle and amplitude of said complex impedance are determined from the measured values in data processing means (20) which is remote from the electrodes; there being a data link (22) between the electronics module and the data processing means.

4. Measuring means for determining the dielectric properties of wood, i.e. its complex impedance comprised of the

resistance $R_x$ and the capacitance $C_x$, comprising a pair of electrodes (16) between which the wood (12) can be disposed, a resistive element (24) connected in series with one of the electrodes, and means (14, 20) adapted to determine the dielectric properties $R_x$ and $C_x$ of the wood disposed between the electrodes by use of the following equations, assuming that the capacitive and resistive components of the wood as complex impedance are in parallel:

$$a = V_2 \cos ! \tag{7}$$

$$b = V_2 \sin ! \tag{8}$$

$$d = \frac{V_1}{(-a(\frac{a}{b})-b)} \tag{9}$$

$$c = \frac{ad}{b} \tag{10}$$

$$C_x = \frac{d}{2\,fR\Pi} \tag{11}$$

$$R_x = \frac{R}{(Hc-1)} \tag{12}$$

where:

$V_1$ is the amplitude of a varying signal of specified voltage to be applied to the electrodes via the resistive element (24)
$V_2$ is the amplitude of the voltage across the electrodes;
! is the phase difference between the voltages $V_1$ and $V_2$,
R is the series resistive element (24);
f is the frequency of the applied voltage;
$C_x$ is the value of the capacitive component of the complex impedance (30); and
$R_x$ is the resistive component of the complex impedance (30).

5. A wood-drying installation which comprises a wood-drying kiln (10) and means arranged for determining the dielectric properties of wood (12) being dried in the kiln, said means comprising a pair of electrodes (16) between which the wood in the kiln can be disposed, means for applying a varying signal of specified voltage to the electrodes (16) via a resistive element (24) connected in series with one of the electrodes (16), and means (14, 20) arranged for determining the amplitude of the applied voltage and the amplitude of the voltage accross the electrodes and the angle representing the phase difference between these signals generating the voltages, said means for determining the dielectric properties making use of the following equations to obtain the dielectric properties of wood, assuming that the capacitive and resistive components of the wood as complex impedance are in parallel:

$$a = V_2 \cos ! \tag{13}$$

$$b = V_2 \sin ! \tag{14}$$

$$d = \frac{V_1}{(-a(\frac{a}{b})-b)} \tag{15}$$

$$c = \frac{ad}{b} \tag{16}$$

$$C_x = \frac{d}{2\,fR\Pi} \tag{17}$$

$$R_x = \frac{R}{(Hc-1)} \tag{18}$$

where:

$V_1$ is the amplitude of the applied voltage;
$V_2$ is the amplitude of the voltage across the electrodes;
! is the phase difference between the voltages $V_1$ and $V_2$,
R is the series resistive element (24);
f is the frequency of the applied voltage;
$C_x$ is the value of the capacitive component of the complex impedance (30); and
$R_x$ is the resistive component of the complex impedance (30).

6. A wood-drying installation as claimed in claim 5, wherein the means for applying a varying signal is adapted to apply a sinusoidal voltage (38) to the electrodes via the resistive element, further comprising means (34..46) adapted for determining the phase angle between the applied voltage $V_1$ and the voltage $V_2$ across the electrodes and the amplitudes of said voltages.

7. A wood-drying installation as claimed in claim 6, wherein the resistive element (24) and means (34...46 )adapted for determining the phase angle between the applied voltage $V_1$ and the voltage $V_2$ across the electrodes and the amplitudes of said voltages form part of an electronics module (14) which is in close proximity to the electrodes and which further comprises data processing means (20) which is remote from the electrodes adapted for determining from the measured values the dielectric properties $R_x$ and $C_x$ of the complex impedance of the wood disposed between the electrodes: there being a data link (22) between the electronics module and the data processing means.

**Patentansprüche**

1. Verfahren zum Bestimmen der dielektrischen Eigenschaften von Holz, d. h. seiner komplexen Impedanz, die aus dem wahren oder reinen Widerstand $R_x$ und der wahren oder reinen Kapazität $C_x$ des Holzes als komplexes Dielektrikum besteht, welches umfasst, das Holz (12) zwischen Elektroden (16) anzuordnen, ein variierendes Signal spezieller Spannung an die Elektroden über ein Widerstandselement (24) anzulegen, das in Reihe mit einer der Elektroden (16) verbunden ist, sowohl die Amplitude der angelegten Spannung als auch die Amplitude der Spannung über den Elektroden zu messen, und durch die gemessene Amplitude der angelegten Spannung, die Amplitude der Spannung über den Elektroden und den Winkel, der die Phasendifferenz zwischen diesen Signalen darstellt, die dielektrischen Eigenschaften $R_x$ und $C_x$ des zwischen den Elektroden angeordneten Holzes durch Verwendung der folgenden Gleichungen unter der Annahme zu bestimmen, dass die Kapazitäts- und Widerstandskomponenten der komplexen Impedanz (30) parallel sind:

$$a = V_2 \cos! \tag{1}$$

$$b = V_2 \sin! \tag{2}$$

$$d = \frac{V_1}{\left(-a\left(\dfrac{a}{b}\right)-b\right)} \qquad (3)$$

$$c = \frac{ad}{b} \qquad (4)$$

$$c_x = \frac{d}{2fR\Pi} \qquad (5)$$

$$R_x = \frac{R}{Hc-1} \qquad (6)$$

wobei:

$V_1$ die Amplitude der anlegten Spannung ist;
$V_2$ die Amplitude der Spannung über den Elektroden ist;
! die Phasendifferenz zwischen den Spannungen $V_1$ und $V_2$ ist;
R das Reihenwiderstandselement (24) ist;
f die Frequenz der angelegten Spannung ist;
$C_x$ der Wert der Kapazitätskomponente der komplexen Impedanz (30) ist; und
$R_x$ die Widerstandskomponente der komplexen Impedanz (30) ist.

2. Verfahren nach Anspruch 1, bei dem die variierende Spannung eine Sinusspannung (38) ist, die an die Elektroden (16) über das Widerstandselement (24) angelegt wird.

3. Verfahren nach Anspruch 2, bei dem die gemessenen Amplituden von $V_1$ und $V_2$ und die Phasendifferenz zwischen $V_1$ und $V_2$ in einem Elektronikmodul (14) bestimmt werden, das sich in dichter Nähe zu den Elektroden befindet, und bei dem der Phasenwinkel und die Amplitude der komplexen Impedanz von den gemessenen Werten in einem Datenverarbeitungsmittel (20) bestimmt werden, das von den Elektroden entfernt ist; wobei ein Datenübermittlungsabschnitt (22) zwischen dem Elektronikmodul und dem Datenverarbeitungsmittel vorhanden ist.

4. Messeinrichtung zum Bestimmen der dielektrischen Eigenschaften von Holz, d. h. seiner komplexen Impedanz, die aus dem Widerstand $R_x$ und der Kapazität $C_x$ besteht, welche ein Paar von Elektroden (16), zwischen denen das Holz (12) angeordnet werden kann, ein Widerstandselement (24), das in Reihe mit einer der Elektroden verbunden ist, und Mittel (14, 20) umfasst, die zum Bestimmen der dielektrischen Eigenschaften $R_x$ und $C_x$ des zwischen den Elektroden angeordneten Holzes durch Verwendung der folgenden Gleichungen ausgelegt sind, wobei angenommen wird, dass die Kapazitäts- und Widerstandskomponenten des Holzes als komplexe Impedanz parallel sind:

$$a = V_2 \cos! \qquad (7)$$

$$b = V_2 \sin! \qquad (8)$$

$$d = \frac{V_1}{\left(-a\left(\dfrac{a}{b}\right)-b\right)} \qquad (9)$$

$$c = \frac{ad}{b} \tag{10}$$

$$c_x = \frac{d}{2fR\Pi} \tag{11}$$

$$R_x = \frac{R}{Hc\text{-}1} \tag{12}$$

wobei:

$V_1$ die Amplitude eines variierenden Signals einer spezifizierten Spannung ist, die an die Elektroden über das Widerstandselement (24) anzulegen ist;
$V_2$ die Amplitude der Spannung über den Elektroden ist;
! die Phasendifferenz zwischen den Spannungen $V_1$ und $V_2$ ist,
R das Reihenwiderstandselement (24) ist;
f die Frequenz der angelegten Spannung ist;
$C_x$ der Wert der Kapazitätskomponente der komplexen Impedanz (30) ist; und
$R_x$ die Widerstandskomponente der komplexen Impedanz (30) ist.

5. Holztrocknungsanlage, die einen Holztrockenofen (10) und Mittel umfasst, die zum Bestimmen der dielektrischen Eigenschaften von Holz (12) eingerichtet sind, welches in dem Ofen getrocknet wird, wobei die Mittel ein Paar Elektroden (16), zwischen denen das Holz in dem Ofen angeordnet werden kann, Mittel zum Anlegen eines variierenden Signals einer spezifischen Spannung an die Elektroden (16) über ein Widerstandselement (24), das in Reihe mit einer der Elektroden (16) verbunden ist, und Mittel (14, 20) umfasst, die zum Bestimmen der Amplitude der angelegten Spannung und der Amplitude der Spannung über den Elektrode und des Winkels eingerichtet sind, der die Phasendifferenz zwischen diesen die Spannungen erzeugenden Signalen darstellt, wobei die Mittel zum Bestimmen der dielektrischen Eigenschaften die folgenden Gleichungen zum Erhalten der dielektrischen Eigenschaften von Holz unter der Annahme verwenden, dass die Kapazitäts- und Widerstandskomponenten des Holzes als komplexe Impedanz parallel sind:

$$a = V_2\cos! \tag{13}$$

$$b = V_2\sin! \tag{14}$$

$$d = \frac{V_1}{\left(-a\left(\frac{a}{b}\right)-b\right)} \tag{15}$$

$$c = \frac{ad}{b} \tag{16}$$

$$c_x = \frac{d}{2fR\Pi} \tag{17}$$

$$R_x = \frac{R}{Hc\text{-}1} \tag{18}$$

wobei:

$V_1$ die Amplitude der anlegten Spannung ist;
$V_2$ die Amplitude der Spannung über den Elektroden ist;

! die Phasendifferenz zwischen den Spannungen $V_1$ und $V_2$ ist,
R das Reihenwiderstandselement (24) ist;
f die Frequenz der angelegten Spannung ist;
$C_x$ der Wert der Kapazitätskomponente der komplexen Impedanz 30) ist; und
$R_x$ die Widerstandskomponente der komplexen Impedanz (30) ist.

6. Holztrocknungsanlage nach Anspruch 5, bei der das Mittel zum Anlegen eines variierenden Signals ausgelegt ist, um eine Sinusspannung (38) an die Elektroden über das Widerstandselement anzulegen, und die ferner Mittel (34..46) umfasst, die zum Bestimmen des Phasenwinkels zwischen der angelegen Spannung $V_1$ und der Spannung $V_2$ über den Elektroden und der Amplituden dieser Spannungen ausgelegt sind.

7. Holztrocknungsanlage nach Anspruch 6, bei der das Widerstandselement (24) und die Mittel (34...46), die zum Bestimmen des Phasenwinkels zwischen der angelegten Spannung $V_1$ und der Spannung $V_2$ über den Elektroden und der Amplituden dieser Spannungen ausgelegt sind, einen Teil eines Elektronikmoduls (14) bilden, das sich in dichter Nähe zu den Elektroden befindet und das ferner ein Datenverarbeitungsmittel (20) umfasst, das von den Elektroden entfernt und ausgelegt ist, um von den gemessenen Werten die dielektrischen Eigenschaften $R_x$ und $C_x$ der komplexen Impedanz des zwischen den Elektroden angeordneten Holzes zu bestimmen: wobei ein Datenübertragungsabschnitt (22) zwischen dem Elektronikmodul und dem Datenverarbeitungsmittel vorgesehen ist.

**Revendications**

1. Procédé de détermination des propriétés diélectriques du bois, à savoir son impédance complexe, comprenant la résistance vraie ou pure résistance $R_x$ et la capacité vraie ou pure capacité $C_x$ du bois en tant que diélectrique complexe, qui comprend le placement du bois (12) à travers les électrodes (16), l'application d'un signal variable d'une tension spécifiée aux électrodes via un élément résistif pour mesurer à la fois l'amplitude de la tension appliquée et l'amplitude de la tension à travers les électrodes, et pour déterminer, d'après l'amplitude mesurée de la tension appliquée, l'amplitude de la tension à travers les électrodes et l'angle représentant la différence de phase entre ces signaux, les propriétés diélectriques $R_x$ et $C_x$ du bois placé entre ces électrodes au moyen des équations suivantes, en supposant que les composantes résistive et capacitive de l'impédance complexe (30) sont en parallèle.

$$a = V_2 \cos 1 \qquad (1)$$

$$b = V_2 \sin 1 \qquad (2)$$

$$d = \frac{V_1}{(-a\frac{(a)}{(b)}-b)} \qquad (3)$$

$$c = \frac{ad}{b} \qquad (4)$$

$$Cx = \frac{d}{2 \, frl} \qquad (5)$$

$$Rx = \frac{R}{lc-1} \qquad (6)$$

où :

$V_1$ est l'amplitude de la tension appliquée ;
$V_2$ est l'amplitude de la tension à travers les électrodes
1 est la différence de phase entre les tensions $V_1$ et $V_2$ ;

X est l'élément résistif en série (24) ;

F est la fréquence de la tension appliquée ;

$C_x$ est la valeur de la composante capacitive de l'impédance complexe (30) ; et

$R_x$ est la composante résistive de l'impédance complexe (30).

2. Procédé selon la revendication 1, dans lequel la tension variable est une tension sinusoïdale (38) appliquée aux électrodes (16) via l'élément résistif (24).

3. Procédé selon la revendication 2, dans lequel l'amplitude mesurée de $V_1$ et $V_2$ et la différence de phase entre $V_1$ et $V_2$ sont déterminées dans un module électronique (34) à étroite proximité des électrodes, et dans lequel l'angle de phase et l'amplitude de ladite impédance complète sont déterminées à partir des valeurs mesurées dans un moyen informatique (20) à distance des électrodes, une liaison de transmission (22) étant en place entre le module électronique et le moyen informatique.

4. Moyen de mesure pour la détermination des propriétés diélectriques du bois, à savoir son impédance complexe comprenant la résistance $R_x$ et de la capacité $C_x$, comprenant une paire d'électrodes (16) entre lesquelles le bois (12) peut être placé, et un élément résistif (24) connecté en série avec l'une des électrodes, et des moyens (14, 20) adaptés pour déterminer les propriétés diélectriques $R_x$ et $C_x$ du bois placé entre les électrodes au moyen des équations suivantes, en supposant que les composantes résistive et capacitive du bois en tant qu'impédance complexe sont en parallèle :

$$a = V_2 \cos 1 \tag{7}$$

$$b = V_2 \sin 1 \tag{8}$$

$$d = \frac{V_1}{(-a\frac{(a)}{(b)}-b)} \tag{9}$$

$$c = \frac{ad}{b} \tag{10}$$

$$Cx = \frac{d}{2\,fRl} \tag{11}$$

$$Rx = \frac{R}{lc-1} \tag{12}$$

où :

$V_1$ est l'amplitude d'un signal variable d'une tension appliquée à appliquer aux électrodes via l'élément résistif (24)

$V_2$ est l'amplitude de la tension à travers les électrodes

1 est la différence de phase entre les tensions V1 et V2

X est l'élément résistif en série (24) ;

f est la fréquence de la tension appliquée ;

$C_x$ est la valeur de la composante résistive de l'impédance complexe (30) ; et

$R_x$ est la composante résistive de l'impédance complexe (30).

5. Installation de séchage de bois qui comprend un four de séchage de bois (11) et des moyens disposés pour déterminer les propriétés diélectriques du bois (12) séché dans ce four, lesdits moyens comprenant une paire d'électrodes (16) entre lesquels peut être placé le bois à l'intérieur du four, un moyen pour appliquer un signal variable d'une tension spécifiée aux électrodes (16) via un élément résistif (24) connecté en série avec l'une des électrodes (16), et des moyens (14, 20) disposés pour déterminer l'amplitude de la tension appliquée et l'amplitude de la tension à travers les électrodes et l'angle représentant la différence de phase entre les signaux générant les tensions, lesdits moyens utilisant l'équation suivante pour déterminer les propriétés diélectriques du bois, en supposant que les composantes capacitive et résistive du bois en tant qu'impédance complexe sont en parallèle :

$$a = V_2 \cos 1 \tag{13}$$

$$b = V_2 \sin 1 \tag{14}$$

$$d = \frac{V_1}{(-a \frac{(a)}{(b)} -b)} \tag{15}$$

$$c = \frac{ad}{b} \tag{16}$$

$$Cx = \frac{d}{2 \, frl} \tag{17}$$

$$Rx = \frac{R}{lc-1} \tag{18}$$

où :

$V_1$ est l'amplitude de la tension appliquée
$V_2$ est l'amplitude de la tension à travers les électrodes
1 est la différence de phase entre les tensions $V_1$ et $V_2$
R est l'élément résistif (24)
f est la fréquence de la tension appliquée
$C_x$ est la valeur de la composante capacitive de l'impédance complexe (30) ; et
$R_p$ est la composante résistive de l'impédance complexe (30)

6. Installation de séchage de bois selon la revendication 5, dans laquelle le moyen pour appliquer un signal variable est adapté pour appliquer une tension sinusoïdale (38) aux électrodes via l'élément résistif, comprenant en outre des moyens (34..46) adaptés pour déterminer l'angle de phase entre la tension appliquée $V_1$ et la tension $V_2$ à travers les électrodes et les amplitudes desdites tensions.

7. Installation de séchage de bois selon la revendication 6, dans laquelle l'élément résistif (24) et les moyens (34...46) adaptés pour déterminer l'angle de phase entre la tension appliquée $V_1$ et la tension $V_2$ à travers les électrodes et les amplitudes desdites tensions font partie d'un module électronique (14) à étroite proximité de l'électronique utilisée et qui comprend en outre un moyen informatique (20) à distance des électrodes adaptées pour déterminer, à partir des valeurs mesurées, les propriétés diélectriques $R_x$ et $C_x$ de l'impédance complexe du bois placé entre les électrodes, une liaison de transmission (22) étant en place entre le module électronique et le moyen informatique.

FIG 1

FIG 2

12

FIG 3

FIG 4